# EUROPEAN PATENT APPLICATION

(11) **EP 4 643 918 A1**
(43) Date of publication of application: **05.11.2025**
(21) Application number: 24747241.8
(22) Date of filing: 22.01.2024
(51) Int. Cl.: A61M 25/00, A61M 25/10

(54) **CATHETER AND CATHETER MANUFACTURING METHOD**

(30) Priority: 26.01.2023 JP 2023010135
(71) Applicant: Terumo Kabushiki Kaisha, Tokyo 151-0072 (JP)
(72) Inventor: MOTOSE, Yuji, Ashigarakami-gun, Kanagawa 259-0151 (JP); KOINUMA, Naoki, Fujinomiya-shi, Shizuoka 418-0015 (JP); SUGIKI, Tsutomu, Ashigarakami-gun, Kanagawa 259-0151 (JP)
(74) Representative: Casalonga
(86) International application number: PCT/JP2024/001590
(87) International publication number: WO 2024/157913

(57) **Abstract**

Provided is a catheter capable of improving followability of a guide wire while maintaining flexibility of a most distal end portion.

A balloon catheter 3 includes an axially extending catheter shaft 310; and a distal end member 320 that is provided on a distal end side of the catheter shaft and is more flexible than the catheter shaft, wherein the distal end member includes a distal end portion 321, an intermediate portion 322, and a proximal end portion 323 in order from a distal end toward a proximal end in the axial direction, and at least a part of the intermediate portion has a harder part 324 than the other parts of the intermediate portion adjacent to a distal end side and a proximal end side of the part, and the distal end portion and the hard part are made of the same material.

## Description

### Technical Field

The present invention relates to a catheter and a method for manufacturing the catheter.

### Background Art

When carrying out various medical procedures inside biological organs, a medical elongated body provided with a catheter shaft (shaft) that includes a hollow member with a lumen, a balloon catheter including a shaft with a guide wire lumen, or the like may be used. In general, in the medical elongated body or the balloon catheter, an elongated shaft and a distal end member disposed on the distal end side of the shaft are provided. The distal end member is formed of a material more flexible than the shaft in order to suppress damage to a body lumen such as a blood vessel.

When a catheter having a flexible distal end member is inserted into a guide wire previously inserted into a curved blood vessel, the distal end member may be deformed to impair followability of the guide wire.

In relation to this, for example, Patent Literature 1 below discloses a catheter in which a shape maintaining body is disposed at the most distal end in order to improve followability of a guide wire.

### Citation List

### Patent Literature

Patent Literature 1: WO 2006/093274 A

### Summary of Invention

### Technical Problem

However, in the above-described catheter, there is a possibility that the most distal end where the hard shape maintaining body is disposed is caught by the stent.

The present invention has been made in view of the above problems, and an object thereof is to provide a catheter capable of improving followability of a guide wire while maintaining flexibility of a most distal end portion.

### Solution to Problem

The above-described object of the present invention is achieved by the following means.

(1) A catheter including:
   a catheter shaft that extends in an axial direction; and
   a distal end member that is provided on a distal end side of the catheter shaft and is more flexible than the catheter shaft, wherein
   the distal end member includes a distal end portion, an intermediate portion, and a proximal end portion in order from a distal end toward a proximal end in the axial direction, and
   at least a part of the intermediate portion has a harder part than the other parts of the intermediate portion adjacent to a distal end side and a proximal end side of the part, and
   the distal end portion and the hard part are made of the same material.
(2) The catheter according to (1), wherein the hard part is located in an interior, in a thickness direction of the distal end member.
(3) The catheter according to (1), wherein the hard part is located on an outer surface and/or an inner surface in a thickness direction of the distal end member.
(4) The catheter according to (3), wherein the hard part is located in an interior, the outer surface, and the inner surface, and an axial length of the hard part in the interior is shorter than an axial length of the hard part located in the outer surface and the inner surface.
(5) The catheter according to (1) or (2), wherein in the hard part, a central part in the axial direction is thick in a thickness direction, and both ends in the axial direction are thin in the thickness direction.
(6) The catheter according to any one of (1) to (5), wherein crystallinity of the hard part is higher than crystallinity of the other parts.
(7) The catheter according to (1), (2), or (5), wherein crystallinity of the hard part is higher at a central part than at both ends in the axial direction.
(8) The catheter according to any one of (1) to (7), wherein an inner surface and an outer surface of the distal end member have lower crystallinity than a part other than the inner surface and the outer surface of the distal end member.
(9) The catheter according to any one of (1) to (8), wherein a distance along the axial direction from a distal end of the distal end member to a distal end of the hard part is longer than a length along the axial direction of the hard part.
(10) The catheter according to any one of (1) to (9), wherein a distance from a distal end of the distal end member to a distal end of the hard part is larger than a radius of the intermediate portion.
(11) The catheter according to any one of (1) to (9), wherein a distance from a distal end of the distal end member to a distal end of the hard part is smaller than a radius of the intermediate portion and larger than an axial length of the distal end portion.
(12) The catheter according to any one of (1) to (9), wherein
   a distance from a distal end of the distal end member to a distal end of the hard part is smaller than a radius of the intermediate portion, and
   a distance from the distal end of the distal end member to a proximal end of the hard part is longer than an axial length of the distal end portion, and
   the axial length of the distal end portion is larger than the distance from the distal end of the distal end member to the distal end of the hard part.
(13) The catheter according to any one of (1) to (12), wherein the distal end portion is configured to have a tapered shape such that an outer diameter gradually decreases toward a distal end side in the axial direction, or configured to have an R-shape such that the outer diameter gradually decreases toward the distal end side in the axial direction.
(14) A catheter including:
   a catheter shaft that extends in an axial direction; and
   a distal end member that is provided on a distal end side of the catheter shaft and is more flexible than the catheter shaft, wherein
   the distal end member includes a distal end portion, an intermediate portion, and a proximal end portion in order from a distal end toward a proximal end in the axial direction, and
   at least a part of the intermediate portion has a harder part than the other parts of the intermediate portion adjacent to a distal end side and a proximal end side of the part, and the distal end portion, the intermediate portion including the harder part, and the proximal end portion of the distal end member are configured as one member of the same material, and
   the hard part is located in an interior, in a thickness direction of the distal end member, and an outer surface and an inner surface of the distal end member are more flexible than the hard part so that the outer surface and the inner surface are more likely to stretch when the distal end member is bent, and
   a hardness of the hard part gradually decreases toward the distal end and the proximal end in the axial direction from a central part of the hard part so that kinking is less likely to occur when the distal end member is bent.
(15) The catheter according to (14), wherein a thickness of the hard part gradually decreases from an interior of the hard part toward the inner surface and the outer surface of the distal end member in the thickness direction of the distal end member.
(16) The catheter according to any one of (1) to (15), including a balloon disposed on an outer periphery of the catheter shaft.
(17) A method for manufacturing a catheter, including:
   disposing a distal end member including a laser light absorbing material in a hollow part of a laser light transmissive elastic body including the hollow part;
   irradiating the distal end member with laser light in a state in which an inner surface of the elastic body is in close contact with an outer surface of the distal end member to heat and melt the distal end member by the laser light absorbing material; and
   cooling after stopping the irradiation of the laser light to form a hard part having high crystallinity on a proximal end side with respect to a distal end of the distal end member.

### Advantageous Effects of Invention

In the catheter configured as described above, since the distal end portion of the distal end member is flexibly configured, the flexibility of the most distal end portion of the distal end member can be maintained. In addition, since the hard part is provided in at least a part of the intermediate portion of the distal end member, followability of the guide wire can be improved.

### Brief Description of Drawings

Fig. 1 is a view illustrating an overall configuration of a balloon catheter according to a first embodiment of the present invention.
Fig. 2 is a view illustrating a cross section along an axial direction of a distal end portion of the balloon catheter according to the first embodiment.
Fig. 3 is a view illustrating a cross section along an axial direction of a distal end member.
Fig. 4 is a view for explaining the method for manufacturing the distal end member of the balloon catheter according to the first embodiment.
Fig. 5 is a view illustrating a cross section along an axial direction of a distal end member according to Modification 1.
Fig. 6 is a view illustrating a cross section along an axial direction of a distal end member according to Modification 2.
Fig. 7 is a view illustrating a cross section along an axial direction of a distal end member according to Modification 3.
Fig. 8 is a view illustrating a cross section along an axial direction of a distal end member according to Modification 4.
Fig. 9 is a view illustrating a cross section along an axial direction of a distal end member according to Modification 5.
Fig. 10 is a view illustrating a cross section along an axial direction of a distal end member according to Modification 6.
Fig. 11 is a view illustrating an overall configuration of a medical elongated body according to a second embodiment of the present invention.
Fig. 12 is a view illustrating a cross section along the axial direction of the distal end portion of the medical elongated body according to the second embodiment.

### Description of Embodiments

### <First Embodiment>

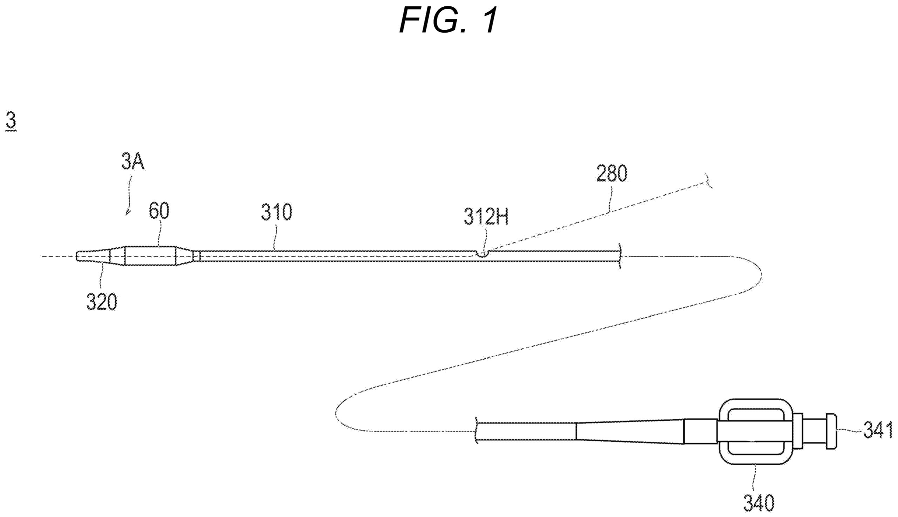

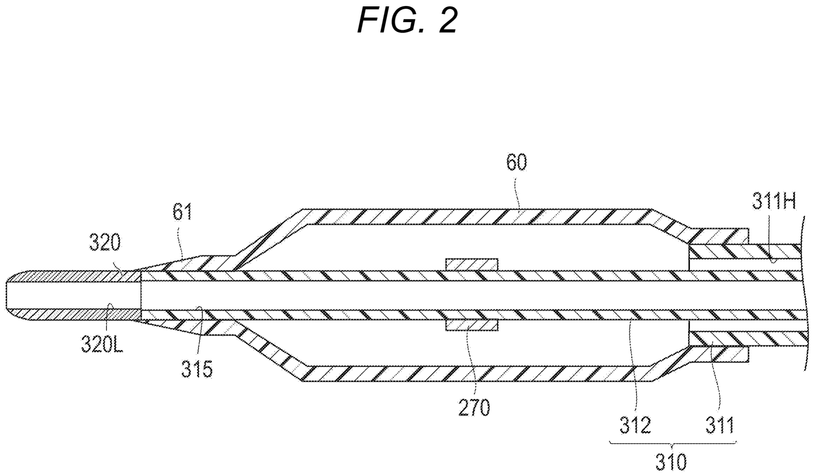

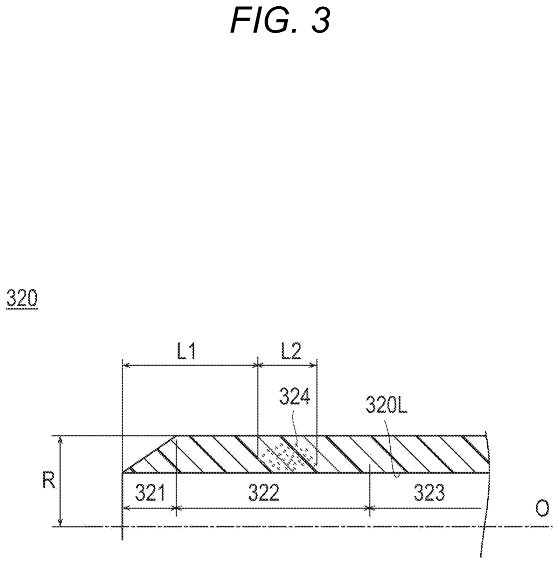

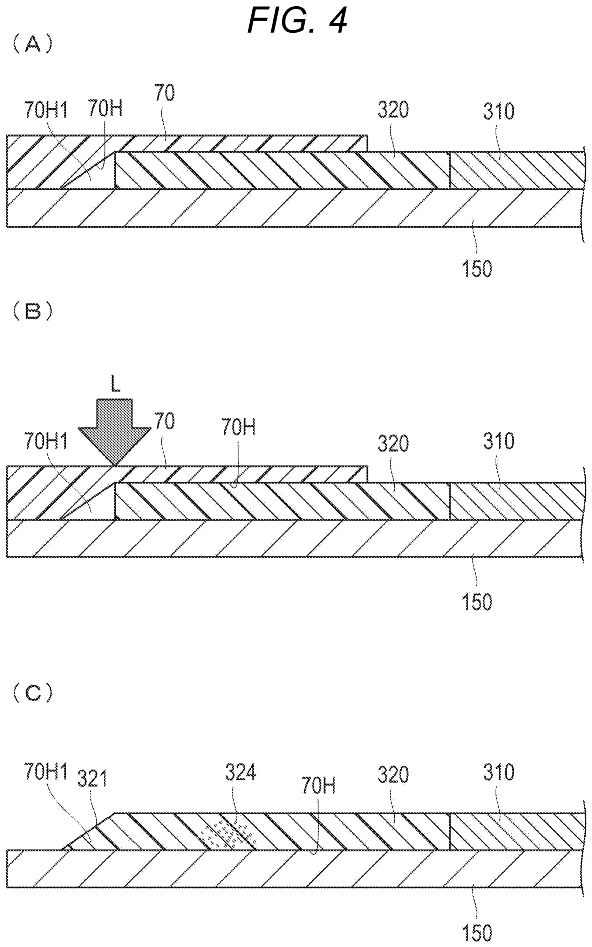

Hereinafter, a balloon catheter 3 according to a first embodiment of the present invention will be described with reference to Figs. 1 to 3. Fig. 1 is a view illustrating an overall configuration of the balloon catheter 3 according to a first embodiment of the present invention. Fig. 2 is a view illustrating a cross section along an axial direction of a distal end portion of the balloon catheter 3 according to the first embodiment. Fig. 3 is a view illustrating a cross section along the axial direction of the distal end member 320 of the balloon catheter 3. In Fig. 3, an upper half of the distal end member 320 is illustrated, and a one-dot chain line indicates a center line. The same applies to Figs. 4 to 10 and 12 described later.

The balloon catheter 3 according to the first embodiment is a medical device used to perform a treatment for a stenosis part by inserting an elongated shaft 310 into a biological organ and expanding a balloon 60 disposed on a distal end side of the shaft 310 at the stenosis part (lesion part) to push and expand the stenosis part.

In the first embodiment, the balloon catheter 3 is configured as a balloon catheter for PTCA expansion used to expand a stenosis part of a coronary artery, and for example, a configuration for the purpose of treating and alleviating a stenosis part developed in a biological organ such as another blood vessel, a bile duct, a trachea, an esophagus, another digestive tract, a urethra, an ear and nose lumen, or another organ can be adopted, and a configuration as a delivery balloon catheter used for the purpose of transporting a medical instrument such as a stent in a living body can be adopted.

In the description of the first embodiment, the part (left side in Fig. 1) of the balloon catheter 3 that is inserted into a living body is referred to as a distal end side, the part of the balloon catheter 3 on which a hub 340 is disposed is referred to as a proximal end side, and the direction in which a shaft 310 of the balloon catheter 3 extends is referred to as an axial direction. A distal end portion represents a certain range including the distal end (the most distal end) and its periphery, while a proximal end portion represents a certain range including the proximal end (the most proximal end) and its periphery. As illustrated in Fig. 3, the direction of the central axis O of the distal end member 320 is referred to as an "axial direction". A direction orthogonal to the central axis O is referred to as a "thickness direction".

As illustrated in Fig. 1, the balloon catheter 3 includes the shaft 310 extending in the axial direction, a distal end member 320 disposed on the distal end side of the shaft 310, the hub 340 disposed on the proximal end side of the shaft 310, and the balloon 60 disposed on the outer periphery of the shaft 310.

As illustrated in Fig. 2, the shaft 310 includes the outer tube shaft 311 provided with the lumen 311H, and the inner tube shaft 312 disposed in the lumen 311H of the outer tube shaft 311.

The shaft 310 has a double tube structure in which an inner tube shaft 312 and an outer tube shaft 311 are disposed with a concentric alignment. In the present embodiment, the balloon catheter 3 is a so-called rapid exchange type in which a proximal end opening (proximal end opening of the inner tube shaft 312) 312H through which the guide wire 280 is guided out is provided near the distal end portion side of the shaft 310. The inner tube shaft 312 and the outer tube shaft 311 may not be disposed with a concentric alignment. That is, the center axis of the inner tube shaft 312 and the center axis of the outer tube shaft 311 may be disposed to be shifted from each other.

As illustrated in Fig. 2, the inner tube shaft 312 has a tubular shape in which a guide wire lumen 315 through which the guide wire 280 is inserted is formed.

A lumen 311H included in the outer tube shaft 311 has a function as a lumen for a pressurizing medium through which a pressurizing medium flows between the outer tube shaft 311 and the inner tube shaft 312.

The hub 340 includes a port 341 connectable in a liquid-tight and air-tight manner to a supplying device such as an indeflator (not illustrated) for supplying a pressurizing medium to the balloon 60. The port 341 of the hub 340 may be, for example, a known Luer taper which a fluid tube or the like can be connected to or separated from. The pressurizing medium (for example, saline, contrast medium, and the like) can flow into the lumen 311H of the outer tube shaft 311 via the port 341 of the hub 340, and is supplied to the balloon 60 through the lumen 311H.

The distal end member 320 is made of a material more flexible than the shaft 310. The distal end member 320 is also referred to as a distal tip, and has a function of reducing damage to a lumen in a body lumen such as a blood vessel, a function of facilitating insertion into a branched blood vessel by following a guide wire that has already been inserted, and furthermore, a function of improving insertability into a stenosis part occurring in the blood vessel.

The inner surface and the outer surface of the distal end member 320 are configured to have crystallinity lower than that of parts other than the inner surface and the outer surface of the distal end member 320. According to this configuration, it is possible to improve the flexibility from the most distal end of the distal end member 320 to the front of the hard part of the intermediate portion 322 described later.

As illustrated in Fig. 3, the distal end member 320 has a distal end portion 321, an intermediate portion 322, and a proximal end portion 323 from the distal end toward the proximal end in the axial direction. In the interior of the distal end member 320, a through-hole 320L is formed to penetrate the distal end member 320 in the axial direction. The through-hole 320L enables a medical device such as a guide wire inserted into the guide wire lumen 315 to be guided out to the distal end side of the medical elongated body 1.

The distal end portion 321 is configured to a tapered shape such that the outer diameter gradually decreases toward the distal end side in the axial direction.

The length of the distal end portion 321 along the axial direction is not particularly limited, but is 0.01 to 20 mm.

A part of the intermediate portion 322 has a harder part 324 than the other parts of the intermediate portion 322 adjacent to the distal end side and the proximal end side of the part (hereinafter referred to simply as "hard part"). The distal end portion 321, the intermediate portion 322, and the proximal end portion 323 of the distal end member 320 are made of the same material. The distal end member 320 including the distal end portion 321, the intermediate portion 322, and the proximal end portion 323 is configured as one member. The crystallinity of the hard part 324 is configured to be higher than the crystallinity of the other parts of the distal end member 320. According to this configuration, as described later, the hard part 324 can be formed by irradiating the laser L, and the hard part 324 can be easily formed. In Fig. 3 and the subsequent figures, the harder part 324 than other parts of the intermediate portion 322 is represented by a large number of dots, but the dots indicate the location of the part by the location of the dots, and each dot does not represent a precipitate or a modified product.

Crystallinity is an index indicating a degree of crystallinity of a resin, and is represented by (crystalline band intensity (absorbance)/amorphous band intensity (absorbance)) × 100 (%) in the present specification. The crystalline band refers to a band that disappears as confirmed by FT-IR measurement at a melting temperature, and the amorphous band refers to a band that does not disappear as confirmed by FT-IR measurement even at a melting temperature. Here, in a case where there are a plurality of crystalline bands and amorphous bands, one crystalline band and one amorphous band are selected as described below. Specifically, one peak with less peak overlap and baseline fluctuation is selected. For example, when the resin is a polyamide resin (resin containing polyamide or polyamide elastomer), and when the intensity at 1161 cm⁻¹ of a plurality of crystalline bands is a peak with less peak overlap and baseline fluctuation as described above, the intensity is taken as the crystalline band intensity. Furthermore, for example, in the same resin as described above, when the intensity at 1369 cm⁻¹ is a peak with less peak overlap and baseline fluctuation as described above among a plurality of amorphous bands, the intensity is taken as the amorphous band intensity. In order to suppress the influence of the variation in the sample thickness, the ratio was calculated as "crystallinity" of the sample as in the formula shown at the beginning with the amorphous band as the denominator and the crystalline band as the numerator.

The crystallinity of the resin is measured as a crystallinity distribution confirmed by imaging IR. In the present specification, the measurement of the crystallinity distribution is carried out under the conditions as follows.
Measurement method: Microscopic-transmission method
Measurement range: 700 µm square (128 pixel square)
Element size: 5.5 µm square
Integration: 128 times
Spectral resolution: 4 cm⁻¹
Pretreatment: A sample is embedded in an epoxy resin and processed with a microtome into a thin section having a thickness of about 10 um.

As illustrated in Fig. 3, the hard part 324 is located in an interior, in the thickness direction of the distal end member 320. The term "interior" as used herein refers to a part inside the outer surface and outside the inner surface in the thickness direction. For example, the hard part 324 is located in the vicinity of the center in the thickness direction of the distal end member 320. According to this configuration, since the inner surface 320A and the outer surface 320B of the distal end member 320 are more flexible than the hard part 324, the influence of contact with the blood vessel is small, and the inner surface 320A and the outer surface 320B of the distal end member 320 easily stretch when the distal end member 320 is bent.

The hardness of the hard part 324 gradually decreases from the interior of the hard part 324 toward the inner surface 320A and the outer surface 320B of the distal end member 320 in the thickness direction of the distal end member 320. According to this configuration, the transition of the physical properties in the radial direction is smooth when the distal end member 320 is bent, and kinking in the distal end member 320 can be prevented. Furthermore, according to the above configuration, the possibility of breakage or the like due to cracks in the distal end member 320 is reduced.

In addition, as illustrated in Fig. 3, the hard part 324 is formed such that a central part in the axial direction is thicker in the thickness direction, and a distal end side and a proximal end side in the axial direction are thinner than the central part in the thickness direction. In other words, as illustrated in Fig. 3, the hard part 324 is configured to have a substantially elliptical shape in the axial direction. At this time, the crystallinity of the hard part 324 is configured such that the central part is higher than both ends in the axial direction. According to this configuration, since the hardness of the hard part 324 gradually changes along the axial direction, kinking hardly occurs when the distal end member 320 is bent.

The hardness of the hard part 324 gradually decreases from the central part toward the distal end in the axial direction. The hardness of the hard part 324 gradually decreases from the central part toward the proximal end in the axial direction. According to this configuration, since the hardness of the hard part 324 gradually changes along the axial direction, kinking is less likely to occur with the hard part 324 as a boundary when the distal end member 320 is bent.

The length of the intermediate portion 322 along the axial direction is not particularly limited, and is, for example, 0.03 to 20 mm. The length of the hard part 324 along the axial direction is not particularly limited, and is, for example, 0.01 to 19.98 mm. The length from the distal end of the intermediate portion 322 to the distal end of the hard part 324 is not particularly limited, but is 0.01 to 19.98 mm.

The proximal end portion 323 is provided on the proximal end side of the intermediate portion 322. The length of the proximal end portion 323 along the axial direction is not particularly limited, but is 0.05 to 20 mm.

As illustrated in Fig. 3, the distance L1 from the distal end of the distal end member 320 to the distal end of the hard part 324 is longer than the length L2 of the hard part 324. According to this configuration, since the flexibility is improved as compared with a configuration in which the distance L1 from the most distal end of the distal end member 320 to the distal end of the hard part 324 is shorter than the length L2 of the hard part 324, when the balloon catheter 3 is inserted into the body, the distal end proceeds to the distal end while being deformed, and it is suppressed that the distal end is caught by the stent or the calcified lesion during the procedure. Note that a configuration in which the distance L1 from the most distal end of the distal end member 320 to the distal end of the hard part 324 is shorter than the length L2 of the hard part 324 is also included in the present invention.

In addition, the distance L1 from the distal end of the distal end member 320 to the distal end of the hard part 324 is longer than the radius R of the intermediate portion 322. According to this configuration, the flexibility is improved, the distal end proceeds to the distal end while being deformed, and it is suppressed from being caught by the stent or the calcified lesion and the hard part 324 is formed, so that it is possible to follow the guide wire.

Examples of the resin forming the distal end member 320 include polyolefins (for example, polyethylene, polypropylene, polybutene, ethylene-propylene copolymer, ethylene-vinyl acetate copolymer, ionomer, or a mixture of two or more types thereof), polyvinyl chloride, polyamide, polyamide elastomer, polyurethane, polyurethane elastomer, and fluororesin. As the resin, one kind of material having high flexibility, good affinity with an adjacent member, and a hardness difference that is not too large may be used singly, or two or more kinds thereof may be used in combination so as to have such characteristics. The resin constituting the distal end member preferably contains a small amount of elastomer, and more preferably contains a polyamide elastomer. The content of the polyamide elastomer in the resin contained in the distal end member is preferably 50% by weight or more, more preferably 80% by weight or more, and still more preferably 100% by weight (consisting of a polyamide elastomer). The polyamide elastomer may be used singly or in combination of two or more types thereof.

The polyamide elastomer is a thermoplastic resin composed of a copolymer having a hard segment derived from a polymer that is crystalline and has a high melting point and a soft segment derived from a polymer that is amorphous and has a low glass transition temperature, the polyamide elastomer having amide bonds (-CONH-) in the polymer backbone forming the hard segment. Constituent units having amide bonds (-CONH-) in the polymer backbone forming the hard segment are also referred to as amide units of the polyamide elastomer.

The "amide units of the polyamide elastomer" in the present specification refers to repeating units derived from amide bonds in the polymer chain of the polyamide elastomer, and the amide units in the polyamide elastomer are preferably at least one of repeating units represented by Formula (1) or Formula (2) below.

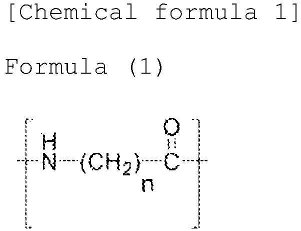

In Formula (1), n is preferably an integer of 2 to 20, and more preferably an integer of 5 to 11.

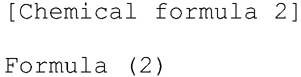

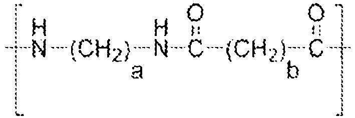

In Formula (2), a is preferably an integer of 4 to 12, b is preferably an integer of 4 to 10, a is more preferably an integer of 11 or 12, and b is more preferably an integer of 4 to 8.

Therefore, the polyamide elastomer preferably contains at least one of the repeating units represented by Formula (1) or Formula (2).

Examples of the polymer forming the soft segment include polyester and polyether. Moreover, examples thereof include polyethers such as polyethylene glycol, polypropylene glycol, polytetramethylene ether glycol (PTMG), and polyester polyol, and ABA-type triblock polyether diols. These can be used singly or in combination of two or more types thereof. Furthermore, a polyether diamine or the like obtained by reacting ammonia or the like with the terminal of polyether can be used, and for example, an ABA-type triblock polyether diamine can be used.

The content of the soft segment in the polyamide elastomer is preferably 1% to 50% by weight and more preferably 10% to 30% by weight.

Among these, the polyamide elastomer is preferably a polyether block amide copolymer in which a polyamide block as a hard segment and a polyether block as a soft segment are bonded via an ester bond.

The distal end member 320 can contain a pigment or dye that develops white, black, blue, red, or yellow, and a mixture thereof. Such a pigment or dye may be selected from materials that absorb laser light and generate heat. Examples of a material that absorbs laser light and generates heat (hereinbelow, also referred to as a laser light-absorbing material) include carbon black, activated carbon, graphite, carbon nanotube, and fullerene; and condensed polycyclic organic pigments such as cyanine-based pigments, nickel dithiolene-based pigments, squarylium-based pigments, naphthoquinone-based pigments, diimmonium-based pigments, azo-based organic pigments, phthalocyanine-based pigments, naphthalocyanine-based pigments, and azulenocyanine-based pigments.

Further, the distal end member 320 can be configured to include a powdered material having high X-ray radiopaque property. Specific examples of the material include compounds of gold, titanium, bismuth, and tungsten.

As illustrated in Fig. 2, the inner tube shaft 312 is provided with an X-ray radiopaque marker 270 indicating the location of the balloon 60 in the axial direction. As the X-ray radiopaque markers 270, for example, a thin metal wire formed of an X-ray radiopaque material, such as a metal such as platinum, gold, silver, iridium, titanium, or tungsten, or an alloy thereof can be used. As the X-ray radiopaque markers 270, a resin material containing powder made of an X-ray radiopaque material may be used.

As illustrated in Fig. 2, the proximal end portion 65 of the balloon 60 is bonded to the distal end portion of the outer tube shaft 311. The distal end portion 61 of the balloon 60 is bonded to the distal end portion of the inner tube shaft 312 and the proximal end portion 323 of the distal end member 320.

The balloon 60 includes an inner layer, an outer layer, and a base layer. Hereinafter, the materials of the inner layer, the outer layer, and the base layer will be described.

The base layer preferably contains a polyamide. In the base layer containing a polyamide, the polyamide accounts for preferably 50% by weight or more, more preferably 80% by weight or more, most preferably 100% by weight (that is, consisting of a polyamide) in a resin constituting the base layer. In a case where polyamide is contained as the resin of the base layer, pressure resistance strength and low compliance characteristics required of the catheter balloon can be ensured, which is preferable. The base layer may contain known additives or high X-ray radiopaque materials as necessary, or may be composed only of polyamide.

As the polyamide, those described in the section of the above-described catheter shaft can be appropriately selected and used.

The polyamide resin can be used singly or in combination of two or more types thereof.

Examples of the additive contained in the base layer as necessary include higher alcohols, hydroxybenzoic acid esters, and aromatic sulfonamides, but are not necessarily limited thereto.

In the cross section perpendicular to the axis at the axial center part of the balloon, the cross-sectional area ratio of the base layer is preferably 30% to 70%, and more preferably 35% to 60%.

The average thickness of the base layer is preferably 5 to 20 µm. The thickness of the base layer may be set to an appropriate thickness from the viewpoint of a balloon diameter, required rupture resistance performance, and passability.

The average thickness of the base layer in a membranous main body constituting the balloon is calculated by conversion with an original tube design dimension and the thickness of the balloon.

The outer layer of the balloon (hereinafter, also referred to as a balloon outer layer) preferably contains an elastomer. Moreover, the inner layer of the balloon (hereinafter, also referred to as a balloon inner layer) and the balloon outer layer preferably contain an elastomer. The elastomer contained in the balloon outer layer preferably includes a polyamide elastomer. In the outer layer containing a polyamide elastomer, the polyamide elastomer accounts for preferably 50% by weight or more, more preferably 80% by weight or more, most preferably 100% by weight (that is, consisting of a polyamide elastomer resin) in a resin constituting the outer layer. In a case where the balloon is attached to the catheter and inserted into a body, with an outer layer containing an elastomer (preferably the polyamide elastomer) formed on the outermost layer, the balloon is flexible and thus excellent in passability through a blood vessel or a body cavity in a living body.

The average thickness of the outer layer in the membranous main body constituting the balloon is preferably 5 to 15 µm, and more preferably 5 to 10 µm. In a case where the average thickness is within a range of 5 to 15 µm, abrasion resistance against a hard component such as a calcified lesion can be improved, and the procedure can be applied more safely.

In the cross section perpendicular to the axis at the axial center part of the balloon, the cross-sectional area ratio of the outer layer is preferably 20% to 50%, and more preferably 25% to 50%.

Within this range, both rupture resistance performance and expansion performance of a hard lesion such as a calcified lesion can be achieved.

The balloon inner layer preferably contains a polyamide elastomer because of good adhesiveness with the base layer and resistance to delamination. Furthermore, in the inner layer containing a polyamide elastomer, the polyamide elastomer accounts for preferably 50% by weight or more, more preferably 80% by weight or more, most preferably 100% by weight (that is, consisting of a polyamide elastomer resin) in a resin constituting the inner layer.

In a case where the inner layer containing a polyamide elastomer is formed on the innermost side, it is possible to provide a catheter balloon exhibiting stable pressure resistance and to impart flexibility to the entire balloon, resulting in ensuring excellent passability in a blood vessel or a body cavity.

The average thickness of the balloon inner layer is preferably 0.1 to 10 µm, and more preferably 0.1 to 7 µm. In a case where the average thickness is within a range of 0.1 to 10 µm, the flexibility of the entire balloon and low compliance characteristics are balanced, which is preferable.

As the polyamide elastomer contained in the inner layer and outer layer of the balloon, the same materials as the polyamide elastomer in the section of the above-described catheter shaft can be used. Thus, the description of the polyamide elastomer will not be repeated herein.

As described above, a preferable aspect of the balloon preferably includes a membranous main body provided with an inner layer that contains a polyamide elastomer and is provided on the innermost side, a base layer that contains a polyamide and is laminated on a surface of the inner layer, and an outer layer that contains a polyamide elastomer and is further provided on the outer side of the base layer. As a result, it is possible to provide the balloon exhibiting the excellent low compliance characteristics without impairing the balance between pressure resistance performance and passability performance.

### <Manufacturing method>

Next, a method for manufacturing the distal end member 320 of the balloon catheter 3 according to the first embodiment of the present invention will be described with reference to Fig. 4.

First, in Fig. 4 (A), the shaft 310 and the distal end member 320 are inserted into the core material 150. As a result, the shaft 310 and the distal end member 320 can move and rotate integrally with the core material 150. The core material is made of, for example, metal. The distal end member 320 is disposed in the hollow part 70H of the elastic body 70. The distal end portion 70H1 of the hollow part 70H of the elastic body 70 has a shape corresponding to the distal end portion 321 of the distal end member 320. In the present description, a space having a truncated cone shape is provided. The elastic body 70 has elasticity and laser transmission properties. The term "laser transmission properties" means that the transmittance of the laser light is 80% or more with respect to a thickness of 1 mm in the radial direction. The elastic body 70 is preferably made of a material having higher heat resistance than the material of the distal end member. Examples of suitable materials include silicone rubber, fluororubber, and a thermoplastic elastomer.

Next, an external force is applied to the distal end member 320 in the radial direction. The external force applying method is not particularly limited, but for example, an external force applying member (not illustrated) including a hollow cylindrical hollow part can be used. According to the external force applying member, similarly to the core material 150 and the elastic body 70, the external force applying member is configured to be rotatably supported with the axial direction as the rotation axis. Then, the elastic body 70 can be inserted into the hollow part of the external force applying member having such a shape.

As described above, by applying an external force using the elastic body 70 and the external force applying member at the time of heating without applying an external force at the time of setting, it is possible to perform highly accurate alignment, and effects such as improvement in quality of fusion and improvement in reproducibility are exerted. In addition, since there is a clearance between the distal end member 320 and the elastic body 70, fusion processing can be performed without affecting the distal end member 320 when the distal end member 320 is inserted into the interior of the elastic body 70.

The shape of the external force applying member is not particularly limited as long as the external force applying member can apply the elastic body in the radial direction. The external force applying member is laser light transmissive similarly to the elastic body 70. The term "laser transmission properties" means that the transmittance of the laser light is 80% or more with respect to a thickness of 1 mm in the radial direction. A material used for the external force applying member may be any material that can apply an external force to the elastic body, and examples thereof include glass, quartz, and sapphire.

Next, the distal end member 320 is irradiated with the laser L in a state where the inner surface of the elastic body 70 is in close contact with the outer surface of the distal end member 320 while the state of applying the external force is maintained (see Fig. 4 (B)). Since the external force applying member and the elastic body 70 have laser transmission properties, the distal end member 320 is directly heated. At the time of heating, the core material 150, the elastic body 70, and the external force applying member may be rotated about the axial direction as a rotation axis to rotate the distal end member 320 about the axial direction as a rotation axis so that heating is uniformly performed.

By such laser light irradiation, the distal end of the distal end member 20 is melted by heat generation of the distal end member 20 itself and heat transfer from the core material 150 to the distal end member 20. As a result, a tapered shape corresponding to the distal end portion 70H1 of the hollow part 70H of the elastic body 70 is formed at the distal end portion 321 of the distal end member 320 (see Fig. 4 (C)). Next, the laser light irradiation is stopped, and the distal end member 320 is rapidly cooled by radiating heat to the surface in contact with the elastic body 70 and the periphery (for example, a core material) from the moment when the distal end member 20 is not heated. This rapid cooling prevents crystallization. On the other hand, a part of the intermediate portion where heat radiation is delayed is crystallized. As a result, the hard part 324 having high crystallinity is formed in the intermediate portion 322 of the distal end member 320.

At the time of laser light irradiation, laser light having a wavelength that causes the distal end of the distal end member 320 to generate heat by radiant heating is emitted. The spot diameter of the laser light can be set to φ0.1 to φ10 mm, and the wavelength of the laser light can be set to 800 to 10,000 nm. As the laser light, a fiber laser (a wavelength of 1070 nm), a YAG laser (a wavelength of 1064 nm), a laser diode (808 nm, 840 nm, 940 nm), or the like can be used. The laser light is substantially irradiated in a direction orthogonal to the axial direction of the distal end member 320. The laser light may be irradiated at an appropriately changed angle.

The output of the laser light can be set to 0.1 to 1300000 W/cm². By setting the output to be relatively small as described above, the heat radiation performance is improved, so that the hard part 324 can be located in the vicinity of the center in the thickness direction of the distal end member 320.

Finally, by releasing the application of the external force to the elastic body 70, the balloon catheter 3 can be taken out.

As described above, the balloon catheter 3 according to the first embodiment includes the shaft 310 extending in the axial direction, and the distal end member 320 provided on the distal end side of the shaft 310 and more flexible than the shaft 310. The distal end member 320 includes a distal end portion 321, an intermediate portion 322, and a proximal end portion 323 in this order from the distal end to the proximal end in the axial direction. At least a part of the intermediate portion 322 has a harder part 324 than other parts of the intermediate portion 322 adjacent to the distal end side and the proximal end side of the part, and the distal end portion 321 and the harder part 324 are made of the same material. According to the balloon catheter 3 configured as described above, since the distal end portion 321 is flexibly configured, the flexibility of the most distal end portion can be maintained. In addition, since the hard part 324 is provided in at least a part of the intermediate portion 322 of the distal end member 320, the followability of the guide wire can be improved by the deformation suppressing effect of the hard part 324.

In addition, the hard part 324 is located in an interior, in the thickness direction of the distal end member 320. According to the balloon catheter 3 configured in this manner, since the inner surface 320A and the outer surface 320B of the distal end member 320 are more flexible than the hard part 324, the influence when coming into contact with the blood vessel is small, and the distal end member 320 is easily stretched when bent.

In the hard part 324, a central part in the axial direction is thick in the thickness direction, and both ends in the axial direction are thin in the thickness direction. According to the balloon catheter 3 configured as described above, since the hardness of the hard part 324 gradually changes along the axial direction, kinking hardly occurs when the distal end member 320 is bent.

In addition, the crystallinity of the hard part 324 is higher than the crystallinity of the other parts. According to the balloon catheter 3 configured as described above, the hard part 324 can be formed by irradiating the distal end member 320 with the laser L while using the same material, there is no concern of detachment when another member is bonded, and the hard part 324 can be easily formed.

In addition, the crystallinity of the hard part 324 is higher at the central part than at both ends in the axial direction. According to the balloon catheter 3 configured as described above, since the hardness of the hard part 324 gradually changes along the axial direction, kinking hardly occurs when the distal end member 320 is bent.

In addition, the inner surface and the outer surface of the distal end member 320 are lower in crystallinity than parts other than the inner surface and the outer surface of the distal end member 320. According to the balloon catheter 3 configured as described above, it is possible to improve the flexibility from the most distal end of the distal end member 320 to the distal end of the hard part of the intermediate portion 322.

In addition, a distance L1 along the axial direction from the distal end of the distal end member 320 to the distal end of the hard part 324 is longer than a length L2 along the axial direction of the hard part 324. According to the balloon catheter 3 configured in this manner,, since the flexibility is improved as compared with a configuration in which the distance L1 from the most distal end of the distal end member 320 to the distal end of the hard part 324 is shorter than the length L2 of the hard part 324, the distal end proceeds to the distal end while being deformed, and it is suppressed that the distal end is caught by the stent or the calcified lesion during the procedure.

In addition, the distance L1 from the distal end of the distal end member 320 to the distal end of the hard part 324 is larger than the radius R of the intermediate portion 322. According to the balloon catheter 3 configured as described above, since the flexibility is improved, the distal end is prevented from proceeding to the distal end while being deformed, and it is suppressed that the distal end is caught by the stent or the calcified lesion.

In addition, the distal end portion 321 is configured to a tapered shape such that the outer diameter gradually decreases toward the distal end side in the axial direction. According to the balloon catheter 3 configured as described above, insertion of the balloon catheter 3 into the living body is facilitated.

In addition, as described above, in the method for manufacturing the balloon catheter 3 according to the first embodiment of the present invention, the distal end member 320 including the laser light absorbing material is disposed in the hollow part 70H of the laser light transmissive elastic body 70 including the hollow part 70H, the distal end member 320 is irradiated with the laser L in a state where the inner surface of the elastic body 70 is in close contact with the outer surface of the distal end member 320, the distal end member 320 is heated and melted by the laser light absorbing material, and is cooled after the irradiation of the laser L is stopped, and the hard part 324 having high crystallinity is formed on the proximal end side with respect to the distal end of the distal end member 320. According to the balloon catheter 3 manufactured by this manufacturing method, since the distal end portion 321 is flexibly configured, the flexibility of the most distal end portion can be maintained. In addition, since the hard part 324 is provided in at least a part of the intermediate portion 322 of the distal end member 320, followability of the guide wire can be improved.

Next, Modifications 1 to 6 of the distal end member 320 described above will be described.

### <Distal end member 420 according to Modification 1>

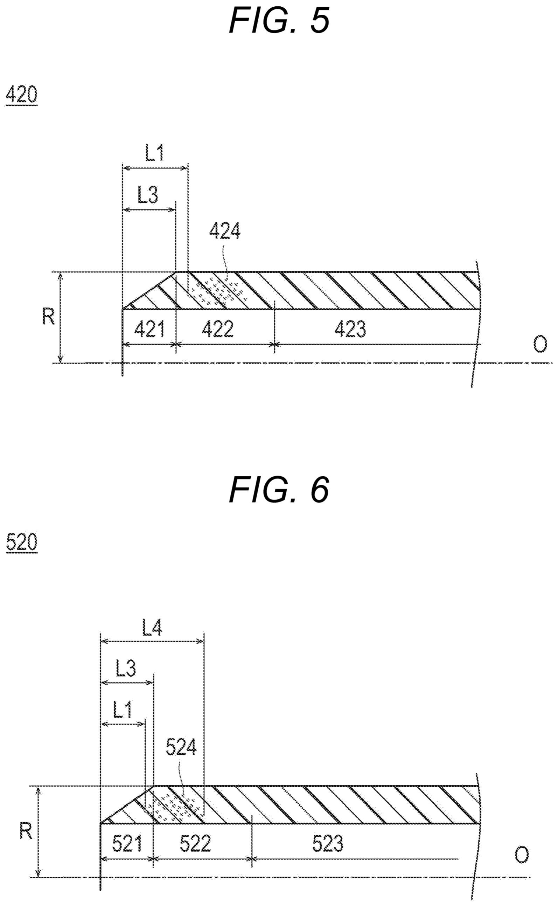

A configuration of a distal end member 420 according to Modification 1 will be described with reference to Fig. 5. Fig. 5 is a view illustrating a cross section along the axial direction of the distal end member 420 according to Modification 1.

As illustrated in Fig. 5, the distal end member 420 according to Modification 1 includes a distal end portion 421, an intermediate portion 422, and a proximal end portion 423 from the distal end toward the proximal end in the axial direction. A hard part 424 having high crystallinity is provided in at least a part of the intermediate portion 422.

In the distal end member 420 according to Modification 1, the distance L1 from the distal end of the distal end member 420 to the distal end of the hard part 424 is smaller than the radius R of the intermediate portion 422. In addition, in the distal end member 420 according to Modification 1, the distance L1 from the distal end of the distal end member 420 to the distal end of the hard part 424 is larger than the axial length L3 of the distal end portion 421. According to this configuration, the distal end of the distal end portion 421 is less likely to be turned up and easily penetrate the stenosis part, and the distal end of the distal end member 420 has high flexibility, so that a load on the blood vessel wall can be reduced.

### <Distal end member 520 according to Modification 2>

A configuration of the distal end member 520 according to Modification 2 will be described with reference to Fig. 6. Fig. 6 is a view illustrating a cross section along the axial direction of the distal end member 520 according to Modification 2.

As illustrated in Fig. 6, the distal end member 520 according to Modification 2 includes a distal end portion 521, an intermediate portion 522, and a proximal end portion 523 from the distal end toward the proximal end in the axial direction. A hard part 524 having high crystallinity is provided in at least a part of the intermediate portion 522.

In the distal end member 520 according to Modification 2, the distance L1 from the distal end of the distal end member 520 to the distal end of the hard part 524 is smaller than the radius R of the intermediate portion 522. A distance L4 from the distal end of the distal end member 520 to the proximal end of the hard part 524 is larger than the axial length L3 of the distal end portion 521. The axial length L3 of the distal end portion 521 is larger than the distance L1 from the distal end of the distal end member 520 to the distal end of the hard part 524. According to this configuration, since the hard part 524 is not provided in the entire region of the distal end portion 521, the flexibility at the distal end of the distal end member 520 can be maintained, and the distal end of the distal end portion 521 is hardly turned up and the part makes it easier to penetrate the stenosis part.

### <Distal end member 620 according to Modification 3>

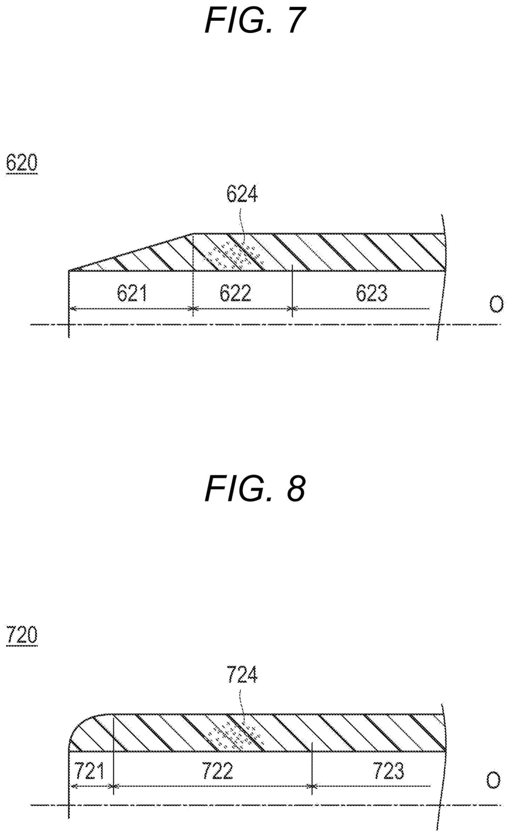

A configuration of the distal end member 620 according to Modification 3 will be described with reference to Fig. 7. Fig. 7 is a view illustrating a cross section along the axial direction of the distal end member 620 according to Modification 3.

As illustrated in Fig. 7, the distal end member 620 according to Modification 3 includes a distal end portion 621, an intermediate portion 622, and a proximal end portion 623 from the distal end toward the proximal end in the axial direction. A hard part 624 having high crystallinity is provided in at least a part of the intermediate portion 622.

The distal end portion 621 of the distal end member 620 according to Modification 3 has a longer length along the axial direction than the distal end portion 321 of the distal end member 320 according to the first embodiment described above.

### <Distal end member 720 according to Modification 4>

A configuration of a distal end member 720 according to Modification 4 will be described with reference to Fig. 8. Fig. 8 is a view illustrating a cross section along the axial direction of the distal end member 720 according to Modification 4.

As illustrated in Fig. 8, the distal end member 720 according to Modification 4 includes a distal end portion 721, an intermediate portion 722, and a proximal end portion 723 from the distal end toward the proximal end in the axial direction. A hard part 724 having high crystallinity is provided in at least a part of the intermediate portion 722.

The distal end portion 721 of the distal end member 720 according to Modification 4 is configured to an R shape such that the outer diameter gradually decreases toward the distal end side in the axial direction.

### <Distal end member 820 according to Modification 5>

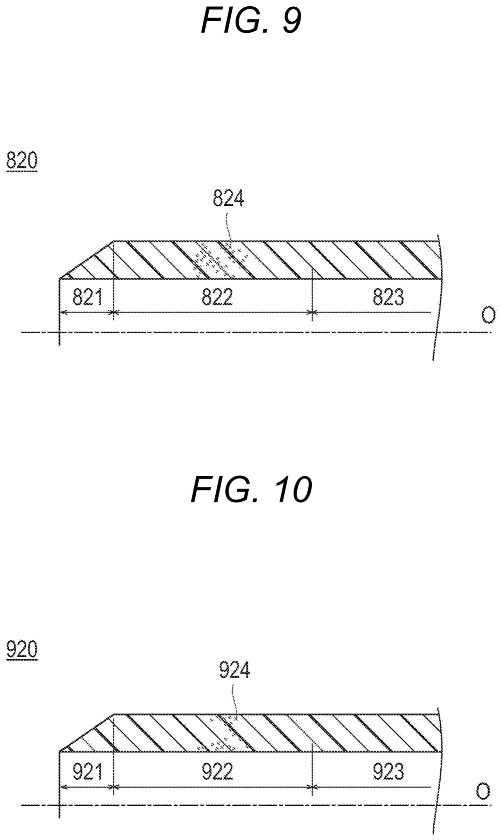

A configuration of a distal end member 820 according to Modification 5 will be described with reference to Fig. 9. Fig. 9 is a view illustrating a cross section along the axial direction of the distal end member 820 according to Modification 5.

As illustrated in Fig. 9, the distal end member 820 according to Modification 5 includes a distal end portion 821, an intermediate portion 822, and a proximal end portion 823 from the distal end toward the proximal end in the axial direction. A hard part 824 having high crystallinity is provided in at least a part of the intermediate portion 822.

As illustrated in Fig. 9, the hard part 824 is uniformly formed along the thickness direction of the distal end member 820.

### <Distal end member 920 according to Modification 6>

A configuration of a distal end member 920 according to Modification 6 will be described with reference to Fig. 10. Fig. 10 is a view illustrating a cross section along the axial direction of the distal end member 920 according to Modification 6.

As illustrated in Fig. 10, the distal end member 920 according to Modification 6 includes a distal end portion 921, an intermediate portion 922, and a proximal end portion 923 from the distal end toward the proximal end in the axial direction. A hard part 924 having high crystallinity is provided in at least a part of the intermediate portion 922.

As illustrated in Fig. 10, the hard part 924 is located on the outer surface and/or the inner surface in the thickness direction of the distal end member 920. In this configuration, when the distal end member 920 hits the calcified stenosis part, stress concentration does not occur on the outer surface and the inner surface, or is dispersed, so that deformation such as turn-up or kinking in the circumferential direction is suppressed. Specifically, the hard part 924 is located on the outer surface and the inner surface in the thickness direction of the distal end member 920. The hard part 324 is located in an interior, in the thickness direction of the distal end member 320. The axial length of the hard part 324 located in an interior, is shorter than the axial length of the hard part 324 located on the outer surface. The axial length of the hard part 324 located in an interior, is shorter than the axial length of the hard part 324 located on the inner surface.

### <Second Embodiment>

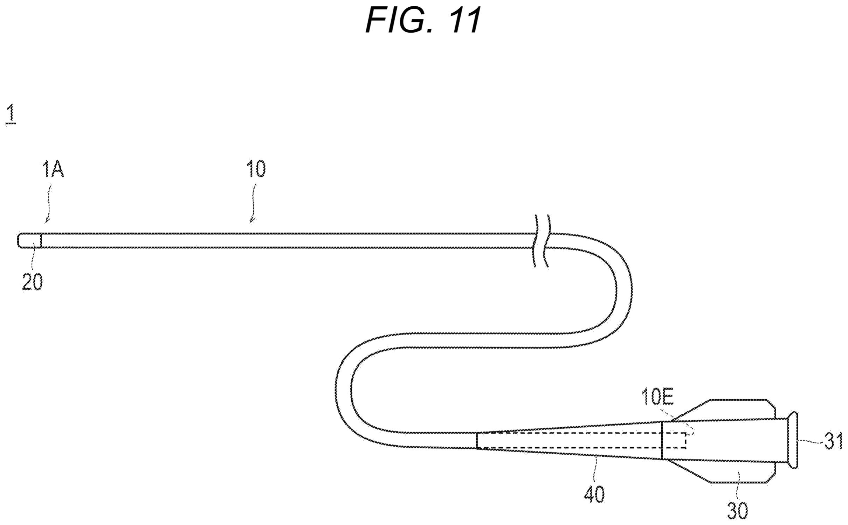

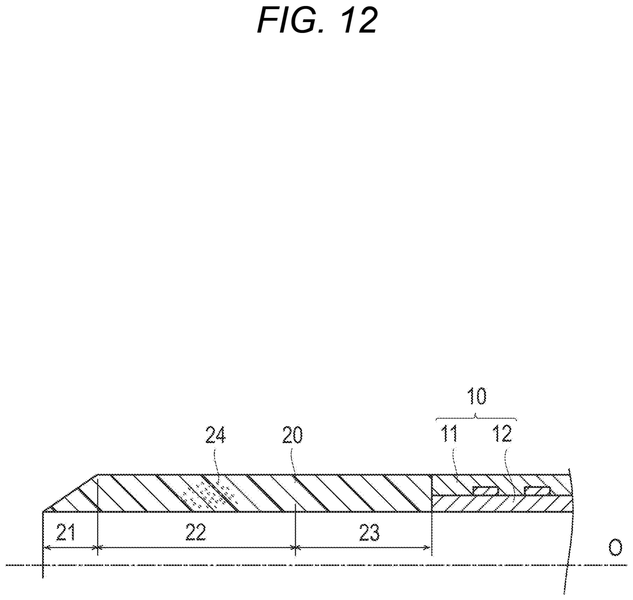

Next, a medical elongated body 1 according to a second embodiment will be described with reference to Figs. 11 and 12. Fig. 11 is a view illustrating an overall configuration of a medical elongated body 1 according to a second embodiment of the present invention. Fig. 12 is a view illustrating a cross section along an axial direction of a distal end portion of a medical elongated body 1 according to a second embodiment.

The medical elongated body 1 is configured as a catheter inserted into a blood vessel, a bile duct, a trachea, an esophagus, a urethra, or other body lumen or body cavity in a living body to perform treatment, diagnosis, or the like.

As illustrated in Figs. 11 and 12, the medical elongated body 1 includes a catheter shaft 10 extending in the axial direction, a distal end member 20 disposed on the distal end side of the catheter shaft 10, a hub 30 disposed on the proximal end side of the catheter shaft 10, and a kink-resistant protector (strain relief) 40 disposed between the catheter shaft 10 and the hub 30.

As illustrated in Fig. 2, the catheter shaft 10 includes an outer layer 11 and an inner layer 12 disposed inward in the radial direction of the outer layer 11. The catheter shaft 10 and the distal end member 20 are bonded to each other.

As illustrated in Fig. 12, the inner layer 12 is continuously formed along the axial direction of the catheter shaft 10. Specifically, the inner layer 12 extends along an inner peripheral surface of the outer layer 11 over substantially the entire length in the axial direction of the catheter shaft 10. For example, it is possible to use the catheter shaft 10 obtained by processing a material forming the inner layer 12 and a material forming the outer layer 11 into a hollow tubular shape having a layer structure by a known method such as co-extrusion molding.

Among them, in a preferred form as the resin constituting the catheter shaft, the resin constituting the outer layer 11 contains a polyamide elastomer, and the resin constituting the inner layer 12 contains a fluororesin. In a more preferred embodiment, the resin constituting the outer layer 11 contains a polyamide elastomer and a polyamide, and the resin constituting the inner layer 12 contains polytetrafluoroethylene. Since such a form is employed, the effect of the outer layer such as improvement in abrasion resistance, protection of the strength layer from external damage, and prevention of pinholes is easily exhibited, and the effect of the inner layer such as securing required pressure resistance strength and compliance is also easily exhibited while securing slidability with the medical instrument passing through the lumen.

Here, as the polyamide and/or the polyamide elastomer, a synthetic product or a commercially available product may be used.

For the inner layer 12 of the catheter shaft 10, a modified polyolefin resin may be used in addition to the fluororesin described above. Examples of the modified polyolefin resin include polyethylene, polypropylene, an α-olefin (for example, 1-butene, 1-hexene, 4-methyl-1-pentene, 1-octene, 1-decene, and the like) polymer, an ethylenepropylene copolymer, a cycloolefin polymer (for example, a cyclic olefin polymer such as norbornene, cyclobutene, or cyclopentene), a cycloolefin copolymer (for example, a copolymer of a cyclic olefin and a chain olefin such as polyethylene, or a copolymer of a cyclic olefin and a diene such as 1,4-hexadiene), and mixtures thereof, which are materials with pendants containing polar and reactive groups; an ethylene-vinyl acetate copolymer. The copolymer has no particular limitation on the structure thereof and may be any one of a random copolymer, an alternating copolymer, a periodic copolymer, or a block copolymer. Among these, the modified polyolefin resin is preferably a modified polyethylene (such as high-density polyethylene, low-density polyethylene, or linear low-density polyethylene) or a modified polypropylene, and more preferably a modified polyethylene, from the viewpoint of the sliding properties of a modified polyolefin layer.

Examples of the polar group and reactivity described above include a carboxyl group, a carboxylic anhydride group, a hydroxyl group, an alkoxy group, an imide group, an acryloyl group, a methacryloyl group, a silanyl group, and/or a silanol group.

As the modified polyolefin resin, a commercially available product may be used, and examples thereof include MODIC (registered trademark) series (H511, H503, L502, L533, L504, M142, M502, M512, M545, A543, A515), LINKLON (registered trademark) series (all of which are manufactured by Mitsubishi Chemical Corporation), Admer (registered trademark) series (manufactured by Mitsui Chemicals, Inc.), and HIMILAN (registered trademark) series (Du Pont-Mitsui Polychemicals Co., Ltd.).

The weight-average molecular weight of the modified polyolefin resin is preferably 1,000 to 10,000,000.

Furthermore, the catheter shaft (specifically, shaft inner layer) can contain a pigment or dye that develops white, black, blue, red, or yellow, and a mixture thereof. Such a pigment or dye may be selected from materials that absorb laser light and generate heat. Examples of a material that absorbs laser light and generates heat (laser light-absorbing material) include carbon black, activated carbon, graphite, carbon nanotube, and fullerene; and condensed polycyclic organic pigments such as cyanine-based pigments, nickel dithiolene-based pigments, squarylium-based pigments, naphthoquinone-based pigments, diimmonium-based pigments, azo-based organic pigments, phthalocyanine-based pigments, naphthalocyanine-based pigments, and azulenocyanine-based pigments. In a suitable aspect, the catheter shaft inner layer contains a laser light-absorbing material and the catheter shaft outer layer is substantially free of the laser light-absorbing material. The blending amount of the laser light-absorbing material is not particularly limited, but for example, 1% to 10% by weight, and may be 2% to 8% by weight in the inner layer. In a case where the laser light-absorbing material is blended in the shaft inner layer, it is preferable to select a transparent shaft outer layer containing the resin described above. Accordingly, the inner layer easily achieves the above-described form.

As necessary, the surface of the outermost layer capable of coming into contact with a living body may be coated with a hydrophilic lubricating material. The hydrophilic lubricating material is not particularly limited as long as it is hydrophilic and has a lubricating property, and a known material can be used. Specific examples thereof include copolymers of epoxy group-containing monomers such as glycidyl acrylate, glycidyl methacrylate, 3,4-epoxycyclohexyl methyl acrylate, 3,4-epoxycyclohexyl methyl methacrylate, β-methyl glycidyl methacrylate, and allyl glycidyl ether with hydrophilic monomers such as N-methylacrylamide, N,N-dimethylacrylamide, and acrylamide; (co)polymers containing the above-described hydrophilic monomer; cellulose-based polymer substances such as hydroxypropyl cellulose and carboxymethyl cellulose; polysaccharides, polyvinyl alcohol, methyl vinyl ether-maleic anhydride copolymers, water-soluble polyamides, poly(2-hydroxyethyl (meth)acrylate), polyethylene glycol, polyacrylamide, polyvinylpyrrolidone, and copolymers of polyvinylpyrrolidone and polyurethane described in US 4,100,309 and JP S59-19582 A. These hydrophilic lubricating materials may be used singly or in the form of a mixture of two or more types thereof.

As illustrated in Fig. 12, the distal end member 20 has a distal end portion 21, an intermediate portion 22, and a proximal end portion 23 from the distal end toward the proximal end in the axial direction. A hard part 24 having high crystallinity is provided in at least a part of the intermediate portion 22. Since the configuration of the distal end member 20 is the same as the configuration of the distal end member 320 of the balloon catheter 3 according to the first embodiment described above, a detailed description thereof will be omitted.

As illustrated in Fig. 11, the hub 30 includes a port 31 that has a function as an insertion port through which a medical device such as a guide wire is inserted into the lumen of the catheter shaft 10. The hub 30 can be attached to cover the outer periphery of a proximal end portion 10E of the catheter shaft 10 using, for example, an adhesive, a fixture (not illustrated), or the like. Furthermore, examples of a constituent material for the hub 30 include a thermoplastic resin such as polycarbonate, polyamide, polysulfone, or polyarylate.

Although the balloon catheter and the medical elongated body according to the present invention have been described above through the embodiments and modifications, the present invention is not limited to only the configuration described in the embodiments, and can be appropriately changed based on the description of the claims.

For example, the medical elongated body described in the second embodiment may not be provided with the kink-resistant protector. Furthermore, the specific use of the medical elongated body is not particularly limited as long as the medical elongated body can be used for the purpose of introducing a medical device (a guide wire, various medical instruments for treatment, or the like) into a living body.

For example, the balloon catheter described in the first embodiment may be configured as a so-called over-the-wire type balloon catheter having a guide wire lumen extending from a distal end to a proximal end of the shaft.

The present application is based on Japanese Patent Application No. 2023-10135 filed on January 26, 2023, the disclosure content of which is incorporated herein by reference in its entirety.

### Reference Signs List

1 Medical elongated body
3 Balloon catheter
10 Catheter shaft
20, 320, 420, 520, 620, 720, 820, 920 Distal end member
21, 321, 421, 521, 621, 721, 821, 921 Distal end portion
22, 322, 422, 522, 622, 722, 822, 922 Intermediate portion
23, 323, 423, 523, 623, 723, 823, 923 Proximal end portion
24, 324, 424, 524, 624, 724, 824, 924 Hard part,
310 Shaft.

## Claims

1. A catheter comprising:
a catheter shaft that extends in an axial direction; and
a distal end member that is provided on a distal end side of the catheter shaft and is more flexible than the catheter shaft, wherein
the distal end member includes a distal end portion, an intermediate portion, and a proximal end portion in order from a distal end toward a proximal end in the axial direction, and
at least a part of the intermediate portion has a harder part than the other parts of the intermediate portion adjacent to a distal end side and a proximal end side of the part, and
the distal end portion and the hard part are made of the same material.

2. The catheter according to claim 1, wherein the hard part is located in an interior, in a thickness direction of the distal end member.

3. The catheter according to claim 1, wherein the hard part is located on an outer surface and/or an inner surface in a thickness direction of the distal end member.

4. The catheter according to claim 3, wherein the hard part is located in an interior, the outer surface, and the inner surface, and an axial length of the hard part in the interior is shorter than an axial length of the hard part located in the outer surface and the inner surface.

5. The catheter according to claim 1 or 2, wherein in the hard part, a central part in the axial direction is thick in a thickness direction, and both ends in the axial direction are thin in the thickness direction.

6. The catheter according to claim 1 or 2, wherein crystallinity of the hard part is higher than crystallinity of the other parts.

7. The catheter according to claim 1 or 2, wherein crystallinity of the hard part is higher at a central part than at both ends in the axial direction.

8. The catheter according to claim 1 or 2, wherein an inner surface and an outer surface of the distal end member have lower crystallinity than a part other than the inner surface and the outer surface of the distal end member.

9. The catheter according to claim 1 or 2, wherein a distance along the axial direction from a distal end of the distal end member to a distal end of the hard part is longer than a length along the axial direction of the hard part.

10. The catheter according to claim 1 or 2, wherein a distance from a distal end of the distal end member to a distal end of the hard part is larger than a radius of the intermediate portion.

11. The catheter according to claim 1 or 2, wherein a distance from a distal end of the distal end member to a distal end of the hard part is smaller than a radius of the intermediate portion and larger than an axial length of the distal end portion.

12. The catheter according to claim 1 or 2, wherein
a distance from a distal end of the distal end member to a distal end of the hard part is smaller than a radius of the intermediate portion, and
a distance from the distal end of the distal end member to a proximal end of the hard part is longer than an axial length of the distal end portion, and
the axial length of the distal end portion is larger than the distance from the distal end of the distal end member to the distal end of the hard part.

13. The catheter according to claim 1 or 2, wherein the distal end portion is configured to have a tapered shape such that an outer diameter gradually decreases toward a distal end side in the axial direction, or configured to have an R-shape such that the outer diameter gradually decreases toward the distal end side in the axial direction.

14. A catheter comprising:
a catheter shaft that extends in an axial direction; and
a distal end member that is provided on a distal end side of the catheter shaft and is more flexible than the catheter shaft, wherein
the distal end member includes a distal end portion, an intermediate portion, and a proximal end portion in order from a distal end toward a proximal end in the axial direction, and
at least a part of the intermediate portion has a harder part than the other parts of the intermediate portion adjacent to a distal end side and a proximal end side of the part, and the distal end portion, the intermediate portion including the harder part, and the proximal end portion of the distal end member are configured as one member of the same material, and
the hard part is located in an interior, in a thickness direction of the distal end member, and an outer surface and an inner surface of the distal end member are more flexible than the hard part so that the outer surface and the inner surface are more likely to stretch when the distal end member is bent, and
a hardness of the hard part gradually decreases toward the distal end and the proximal end in the axial direction from a central part of the hard part so that kinking is less likely to occur when the distal end member is bent.

15. The catheter according to claim 14, wherein a thickness of the hard part gradually decreases from an interior of the hard part toward the inner surface and the outer surface of the distal end member in the thickness direction of the distal end member.

16. The balloon catheter according to claim 1 or 14, comprising a balloon disposed on an outer periphery of the catheter shaft.

17. A method for manufacturing a catheter, comprising:
disposing a distal end member including a laser light absorbing material in a hollow part of a laser light transmissive elastic body including the hollow part;
irradiating the distal end member with laser light in a state in which an inner surface of the elastic body is in close contact with an outer surface of the distal end member to heat and melt the distal end member by the laser light absorbing material; and
cooling after stopping the irradiation of the laser light to form a hard part having high crystallinity on a proximal end side with respect to a distal end of the distal end member.
